## Europäisches Patentamt

## European Patent Office

## Office européen des brevets

(19)

(11) Veröffentlichungsnummer: **0 204 192**
**B1**

(12) **EUROPÄISCHE PATENTSCHRIFT**

(45) Veröffentlichungstag der Patentschrift:
09.08.89

(51) Int. Cl.⁴: **A61B 8/02**, G01S 7/52,
G01S 15/50, G01S 15/66

(21) Anmeldenummer: 86106631.4

(22) Anmeldetag: 15.05.86

(54) Schaltungsanordnung zum Erfassen der Herzschlagsbewegung.

(30) Priorität: 25.05.85 DE 3518967

(43) Veröffentlichungstag der Anmeldung
10.12.86 Patentblatt 86/50

(45) Bekanntmachung des Hinweises auf die Patenterteilung:
09.08.89 Patentblatt 89/32

(84) Benannte Vertragsstaaten:
DE FR GB NL

(56) Entgegenhaltungen:
EP-A- 0 027 215
DE-B- 2 617 158
US-A- 3 802 253
US-A- 3 991 365
US-A- 4 143 650

MEDICAL & BIOLOGICAL ENGINEERING &
COMPUTING, Band 19, Nr. 1, Januar 1981, Seiten 1-10;
A.J. COUSIN: "On processing of Doppler returns from
the foetal heart: an electronic processing method for
extraction of valvular timing information"
M.I. SKOLNIK: "Introduction to Radar Systems", 1962,
Seiten 189-190, McGraw-Hill Book Co., Inc., New York,
US;

(73) Patentinhaber: Hewlett-Packard GmbH, Herrenberger
Strasse 110, D-7030 Böblingen(DE)

(72) Erfinder: Schulenberg, Andreas, Dipl.-Ing.,
Adalbert-Stifter-Weg 2, D-7036 Schönaich(DE)
Erfinder: Schraag, Martin, Dipl.-Ing.,
Karl-Pfitzer-Strasse 21, D-7032 Sindelfingen(DE)

(74) Vertreter: Schulte, Knud, Dipl.-Ing., c/o Hewlett-Packard
GmbH Europ. Patent- und Lizenzabteilung
Postfach 1430 Herrenberger Strasse 130,
D-7030 Böblingen(DE)

(56) Entgegenhaltungen: (Fortsetzung)
IEEE TRANSACTIONS ON BIOMEDICAL ENGINEERING,
Band BME-30, Nr. 9, September 1983, Seiten 577-584,
IEEE, New York, US; A.J. COUSIN et al.: "A tracking
system for pulsed ultrasound images: application to
quantification of fetal breathing movements"

# Beschreibung

Die Erfindung betrifft eine Schaltungsanordnung zum Erfassen der Herzschlagsbewegung, insbesondere der fötalen Herzschlagsbewegung, gemäß dem Oberbegriff von Patentanspruch 1.

Eine Schaltungsanordnung zum Erfassen der fötalen Herzschlagsbewegung ist bekannt aus US-PS 4 143 650. Zum Erfassen der Herzschlagsbewegung wird die bekannte Schaltungsanordnung mit einem Sender und mit einem Empfänger für Ultraschall verbunden, die von außen auf den Körper einer Schwangeren aufgesetzt werden. Die von dem Sender ausgesandten Ultraschallsignale einer bestimmten Trägerfrequenz werden von Strukturen innerhalb des Körpers reflektiert und die reflektierten und vom Empfänger aufgenommenen Signale werden in elektrische Signale umgewandelt, die in der angeschlossenen Schaltungsanordung ausgewertet werden. Ultraschallsignale, die an bewegten Strukturen, beispielsweise am fötalen Herzen, reflektiert werden, sind aufgrund des Doppler-Effektes gegenüber den Sendesignalen in ihrer Frequenz verschoben. Die frequenzverschobenen Echosignale werden in der angeschlossenen Schaltungsanordnung demoduliert, so daß sich das der Geschwindigkeit der Herzschlagsbewegung entsprechende Dopplersignal ergibt, aus dem die Frequenz des fötalen Herzschlages abgeleitet werden kann.

Bei der bekannten Schaltungsanordnung können unter Berücksichtigung der Ausbreitungsgeschwindigkeit der Ultraschallsignale im Körper der Schwangeren verschiedene Tiefenbereiche derart eingestellt werden, daß jeweils nur die aus diesem Tiefenbereich stammenden Echosignale ausgewertet werden. Auf diese Weise können Störsignale unterdrückt werden, die von sich bewegenden Schichten oder Organen herrühren, die sich in einem anderen als dem eingestellten Tiefenbereich befinden. Der Tiefenbereich wird dabei von einer Bedienungsperson entsprechend der vermuteten Tiefe des fötalen Herzens eingestellt.

Wenn die Bedienungsperson keine Kenntnis über die momentane Tiefe des fötalen Herzens hat, müßte der Tiefenbereich in einem zeitaufwendigen und umständlichen Probierverfahren eingestellt werden, bei welchem in aufeinanderfolgenden Suchschritten unterschiedliche Tiefenbereiche eingestellt werden müßten, und schließlich diejenige Einstellung zu wählen wäre, bei der sich nach Einschätzung der Bedienungsperson das beste Meßsignal ergibt. Nachteilig dabei wäre neben dem hohen Zeitaufwand zur Einstellung der richtigen Tiefe, daß während des Einstellungsvorganges das Herzschlagssignal verloren gehen kann, wenn ein Tiefenbereich gewählt wird, der das fötale Herz nicht enthält.

Weiterhin kann sich infolge der häufig vorkommenden Lageänderungen des Fötus das fötale Herz während einer Untersuchung aus dem einmal eingestellten Teifenbereich herausbewegen, so daß eine zuverlässige Messung der Herzschlagsfrequenz nicht mehr möglich ist. Da die Bedienperson keine Information darüber hat, in welche Richtung sich bei einer solchen Lageänderung das fötale Herz bewegt hat, müßte zur Neueinstellung des Tiefenbereiches auch in diesem Falle das erwähnte aufwendige Probierverfahren angewandt werden.

Aus US-A 3 802 253 ist eine Meß- und Aufzeichnungsvorrichtung bekannt, bei welcher Ultraschallimpulse in den Körper einer Versuchsperson ausgesandt und die an inneren Organen, beispielsweise dem Herzen, reflektierten Ultraschallsignale empfangen und dargestellt werden. Die bekannte Vorrichtung enthält eine Torschaltung, die es ermöglicht, die Tiefe der untersuchten Echos bzw. die Zeitverzögerung relativ zu den ausgesandten Ultraschallimpulsen auszuwählen und nachzuregeln. Bei der bekannten Vorrichtung wird kein Gebrauch von Dopplersignalen gemacht.

Eine Schaltungsanordnung zum Erfassen der Herzschlagsbewegung gemäß dem Oberbegriff von Patentanspruch 1 ist bekannt aus DE-B 2 617 158. Dieser nach dem Ultraschall-Doppler-Prinzip arbeitende Herzfrequenzmesser weist zwei mit einem Ultraschallempfänger verbundene Signalverarbeitungskanäle zur Erfassung von Dopplersignalanteilen aus der Herzbewegung getrennt nach beiden Herzpulsationsrichtungen auf. Die beiden Signalverarbeitungskanäle umfassen jeweils eine Mischstufe, die von je einem zweier um 90° phasenverschobener Signale der Ultraschallträgerfrequenz gespeist sind, einen Dopplersignaldemodulator, einen Integrator, eine Sample-and-Hold-Schaltung und ein Bandpaßfilter. Die Ausgangssignale der Bandpaßfilter werden einem Phasenkomparator zugeführt. Dessen Ausgangssignal enthält damit Anteile aus der Hin- und der Wegbewegung der Herzwand, wobei die Signalanteile für die eine Bewegungsrichtung positiv und für die andere Bewegungsrichtung negativ sind. Eine nachfolgende Schaltung selektiert dann zur Ableitung der Herzfrequenz den Signalanteil mit der höchsten Amplitude.

Demgegenüber liegt der Erfindung die Aufgabe zugrunde, eine Schaltungsanordnung gemäß dem Oberbegriff von Patentanspruch 1 anzugeben, welche auch bei sich bewegendem Fötus eine schnellere und zuverlässigere Einstellung des Tiefenbereiches des fötalen Herzens ermöglicht.

Erfindungsgemäß wird diese Aufgabe gelöst durch die kennzeichnenden Merkmale von Patentanspruch 1.

Ein Grundgedanke der Erfindung geht aus von der Erkenntnis, daß die Einstellung des Tiefenbereiches beschleunigt werden kann durch Ableiten einer Tendenzanzeige, die angibt, in welcher Richtung sich das fötale Herz befindet. Dazu sind wenigstens zwei Empfangskanäle zum Auswerten der reflektierten Ultraschallsignale vorgesehen, die jeweils zum Ableiten des der Herzschlagsbewegung entsprechenden Dopplersignales freigegeben werden, so daß die in den beiden Kanälen ausgewerteten Signale Echosignalen aus verschiedenen Tiefenbereichen entsprechen. In jedem Kanal wird in aufeinanderfolgenden Herzschlagsperioden jeweils die Amplitude der dem Herzschlag entsprechenden Dopplersignale abgeleitet. Aus den während einer Herzschlagsperiode festgestellten Amplituden der Dopplersignale in unterschiedlichen Kanälen ergibt sich unmittelbar die Richtung, in welcher sich das fötale Herz befindet. In der folgenden Herzschlagsperiode werden dann die beiden Tiefenbereiche in die in der vorhergehenden Herzschlagsperiode bestimmten Richtung verschoben, indem die Zeitsteuerung der ausgesandten Ultraschallimpulse relativ zu den Empfangsfenstern in den Empfangskanälen verändert wird, d.h. indem die Dauer und der zeitliche Abstand zwischen ausgesandten Ultraschallimpulsen und Empfangsfenstern entsprechend eingestellt werden. Falls erforderlich, werden die Tiefenbereiche verkleinert, indem entweder der ausgesandte Ultraschallimpuls oder das Empfangsfenster verkürzt werden. Auf diese Weise wird in wenigen Schritten die Tiefe des Fötalen Herzens festgestellt und der Tiefenbereich entsprechend eingestellt, so daß die empfangenen Echosignale einen wesentlich geringeren Anteil von Störsignalen enthalten. Die jeweils während eines Suchschritts untersuchten Tiefenbereiche überlappen sich dabei, so daß während des Suchvorganges stets ein zur Herzschlagsbestimmung auswertbares Nutzsignal zur Verfügung steht und stets eine nutzbare Richtungsanzeige für die Tiefe des Herzens abgeleitet werden kann.

Die Erfindung hat den weiteren Vorteil, daß Langzeituntersuchungen des fötalen Herzschlages durchgeführt werden können, ohne daß ein Bediener anwesend sein muß, der bei Lageänderungen des Fötus den Tiefenbereich nachzustellen hätte. Gemäß der Erfindung kann während der Messung laufend die Tiefe des fötalen Herzens bestimmt und der Tiefenbereich durch Einstellung der Zeitsteuerung der Sendeimpulse und Empfangsfenster entsprechend nachgestellt werden, so daß auch bei Bewegungen des Fötus stets eine optimale Signalqualität aufrechterhalten wird.

Weitere vorteilhafte Ausgestaltungen der Erfindung sind in den übrigen Unteransprüchen angegeben.

Nachfolgend wird ein Ausführungsbeispiel der Erfindung anhand der Zeichnung näher erläutert. In der Zeichnung zeigen:

Figur 1: schematisch die Anordnung eines Ultraschallwandlers auf dem Abdomen einer Schwangeren und die Lage des zu untersuchenden fötalen Herzens,

Figur 2: schematisch ein Blockschaltbild eines Ausführungsbeispieles der Erfindung,

Figur 3: eine Schaltung zum Erzeugen von Steuersignalen für den Ultraschallsender und für die Empfangsschaltungen in einer Vorrichtung nach der Erfindung,

Figuren 4a bis 4i: den zeitlichen Verlauf von Steuersignalen, die beim Betrieb der Vorrichtung gemäß Figur 2 auftreten,

Figuren 5a bis 5k: ein weiteres Beispiel für Steuersignale, die beim Betrieb der Vorrichtung gemäß Figur 2 auftreten,

Figur 6: ein Flußdiagramm, welches das Suchverfahren zum Bestimen der Tiefe des fötalen Herzens veranschaulicht, und

Figuren 7a bis 7c: den zeitlichen Verlauf von Steuersignalen für den Ultraschallsender und für den Ultraschallempfänger sowie die sich daraus ergebende Empfindlichkeit in der Vorrichtung nach Figur 2 nach dem Abschluß des Suchvorganges.

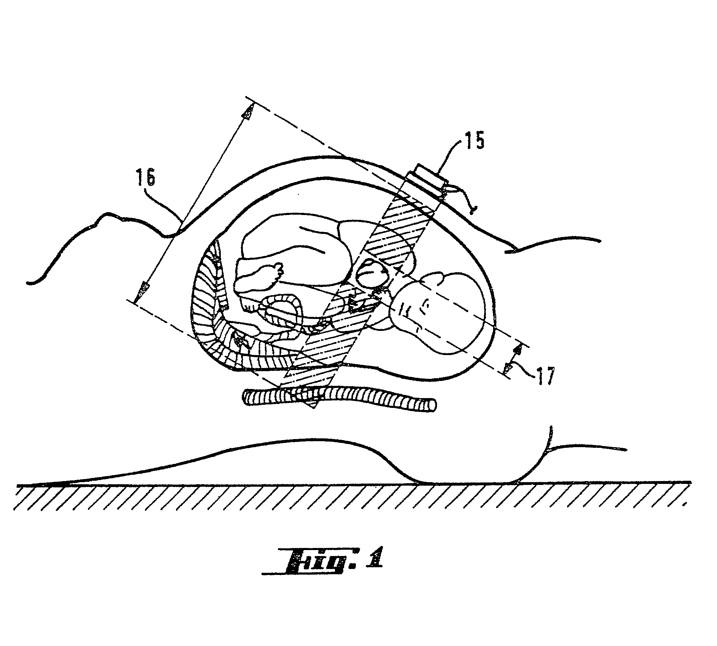

Gemäß Figur 1 wird zum Untersuchen des fötalen Herzschlages auf dem Abdomen der Schwangeren ein Ultraschallwandler 15 angeordnet, der einen Ultraschallstrahl in Richtung auf das fötale Herz aussendet. Der Strahl wird an verschiedenen Schichten im Körper der Schwangeren und des Kindes reflektiert, unter anderem am fötalen Herzen. Während bei bekannten Vorrichtungen zur Herzschlagsmessung reflektierte Ultraschallsignale aus dem gesamten Bereich 16 oder aus einem von einer Bedienperson fest eingestellten Bereich innerhalb des Körpers der Schwangeren empfangen und ausgewertet werden, ermöglicht die Erfindung die automatische Einschränkung des zu untersuchenden Bereiches auf die Region 17 des fötalen Herzens.

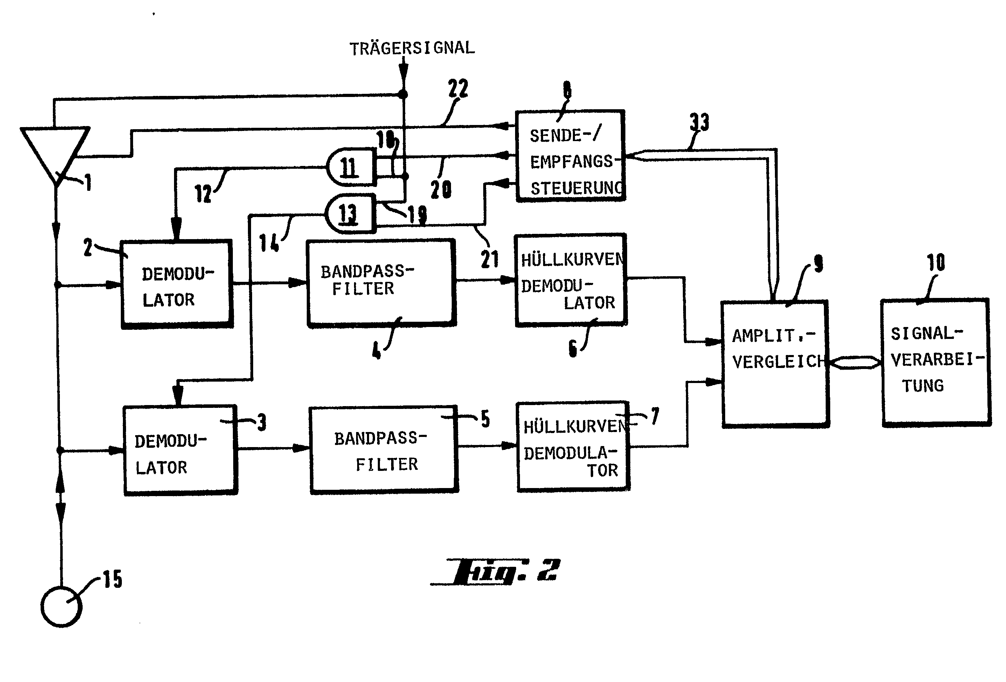

Gemäß Figur 2 ist die erfindungsgemäße Schaltungsanordnung mit einem Ultraschallwandler 15 verbindbar, der einen oder mehrere piezoelektrische Kristalle enthalten kann und der von einer Treiberschaltung 1 zum Aussenden von Ultraschallimpulsen angeregt wird. Zum Ausführen einer fötalen Herzuntersuchung wird der Ultraschallwandler 15 auf den Körper der Schwangeren aufgesetzt, so daß Ultraschallsignale hoher Frequenz, nachfolgend als Trägersignale bezeichnet, in das Körperinnere eintreten können. Die von verschiedenen Schichten innerhalb des Körpers reflektierten Ultraschallsignale, einschließlich der von dem fötalen Herzen reflektierten Signale, werden von einem außen auf dem Abdomen der Schwangeren angeordneten Ultraschallempfänger aufgenommen und in elektrische Ausgangssignale umgewandelt. Aus den an dem bewegten fötalen Herzen reflektierten und aufgrund des Dopplereffektes frequenzverschobenen Ultraschallsignalen wird in der angeschlossenen Auswerteschaltung ein dem Herzschlag entsprechendes Signal abgeleitet. Das dem fötalen Herzschlag entsprechende Signal rührt dabei im allgemeinen von Herzmuskel-, Herzklappen- und zugehörigen Blutströmungsbewegungen des Fötus her. Das die Information über den Herzschlag enthaltende Signal stellt aber nur einen sehr geringen Anteil des gesamten reflektierten Signales dar, in welchem Reflexionssignale von nicht oder schwach bewegten Trennflächen mit entsprechender Frequenzverschiebung den Hauptanteil bilden.

Als Empfänger wird vorzugsweise derselbe Kristall verwendet, der auch als Ultraschallquelle benutzt wird. In diesem Falle wird dieser Sende-/Empfangskristall in aufeinanderfolgenden Sende-/Empfangsperioden jeweils derart angesteuert, daß er während eines ersten Teils einer Periode als Sender für Ultraschallwellen und während eines zweiten Teiles als Empfänger arbeitet. Der Ultraschallwandler 15 im dargestellten Ausführungsbeispiel wird zwar sowohl zum Aussenden als auch zum Empfangen von Ultraschallsignalen benutzt, es wäre jedoch ebenso möglich, verschiedene Kristalle als Sender und als Empfänger zu verwenden.

Die elektrischen Ausgangssignale des Ultraschallempfängers 15 werden zwei Signalverarbeitungskanälen zugeführt, die im folgenden als Nutzkanal bzw. als Vergleichskanal bezeichnet werden. Da die beiden Kanäle schaltungstechnisch im wesentlichen identisch sind, wird nachfolgend nur der Nutzkanal ausführlich beschrieben. Das Ausgangssignal des Ultraschallwandlers 15 wird im Nutzkanal einem Demodulator 2 zugeführt, welcher Ausgangssignale mit Frequenzanteilen erzeugt, die der Differenz der Frequenzen des Trägersignales und des dopplerverschobenen reflektierten Ultraschallsignales entsprechen. Zum Erzeugen dieser Dopplersignale wird einem anderen Eingang des Demodulators 2 auf einer Leitung 12 das gepulste Trägersignal zugeführt, das die gleiche Grundfrequenz wie das Sendesignal enthält, und im Demodulator 2 mit den Ausgangssignalen des Ultraschallwandlers 15 gemischt. Die Zuführung der Trägersignale wird von einer Sende-Empfangssteuerschaltung 8 mittels einer Torschaltung 11 so gesteuert, daß das Dopplersignal nur während eines wählbaren Zeitintervalles erzeugt wird. Einzelheiten des zeitlichen Verlaufes dieser Steuerung sind weiter unten erläutert. Die Torschaltung 11 ist vorzugsweise als UND-Gatter ausgebildet.

Mit dem Ausgang des Demodulators 2 ist ein Bandpaßfilter 4 verbunden, welches nur solche Signale durchläßt, deren Frequenzen im ungefähr zu erwartenden Frequenzbereich der Dopplersignale liegt. Bei einer Trägerfrequenz der Ultraschallsignale von etwa 1 MHz kann beispielsweise ein Bandpaßfilter mit Eckfrequenzen von 100 Hz bzw. 475 Hz verwendet werden. Das Ausgangssignal des Bandpaßfilters 4 wird einem Hüllkurvendemodulator 6 zum Erzeugen eines Hüllkurvensignales zugeführt, dessen Signalform der Einhüllenden des Signalverlaufes des jeweiligen Dopplersignales entspricht. Die Einhüllende der Dopplersignale entspricht im wesentlichen der Intensitätsänderung der frequenzverschobenen Echosignale aufgrund der fötalen Herzschlagsbewegung, so daß die Spitzenwerte des Ausgangssignales des Hüllkurvendemodulators im Rhythmus des fötalen Herzschlages auftreten.

Der ebenfalls mit dem Ultraschallwandler 15 verbundene Vergleichskanal weist einen Demodulator 3, ein Bandpaßfilter 5 und einen Hüllkurvendemodulator 7 auf, die im wesentlichen mit den Einrichtungen des Nutzkanales übereinstimmen. Dem Demodulator 3 wird auf einer Leitung 14 das Trägersignal zum Erzeugen eines Dopplersignales zugeführt, allerdings unterscheidet sich das Zeitintervall, während dessen das Trägersignal zugeführt wird, von dem entsprechenden Zeitintervall im Nutzkanal.

Die Amplituden der Hüllkurvensignale im Nutzkanal und im Vergleichskanal werden anschließend in einer Amplitudenvergleichsschaltung 9 miteinander verglichen, um ein Ausgangssignal zu erzeugen, welches der Sende-/Empfangssteuerschaltung 8 zugeführt wird, die in Abhängigkeit von diesem Signal die Zuführung der Trägerfrequenzsignale auf den Leitungen 12 bzw. 14 zu den Demodulatoren 2 bzw. 3 steuert. Der Amplitudenvergleich kann mittels einer Analogschaltung durchgeführt werden, vorzugsweise wird er jedoch digital durchgeführt, wozu die Hüllkurvensignale im Nutzkanal und im Vergleichskanal vorher mit Hilfe von Analog/Digital-Wandlern (nicht dargestellt) digitalisiert werden. Bei einer digitalen Signalverarbeitung wird vorzugsweise ein Mikroprozessor zum Amplitudenvergleich und zum Erzeugen eines entsprechenden Steuersignales verwendet, welches auf einer Leitung 18 der Sende-/Empfangssteuerschaltung 8 zugeführt wird.

In einer Signalverarbeitungseinrichtung 10 wird aus dem zeitlichen Abstand zweier Spitzenwerte des (gegebenenfalls digitalisierten) Ausgangssignales einer der beiden Hüllkurvendemodulatoren die Periode bzw. die Frequenz des fötalen Herzschlages bestimmt.

Im folgenden wird die Steuerung der Zuführung der Trägersignale durch die Sende-/Empfangssteuerschaltung 8 zu den Demodulatoren 2 bzw. 3 im Nutzkanal bzw. im Vergleichskanal näher beschrieben:

Die Sende-/Empfangssteuerschaltung 8 steuert die Sendetreiberschaltung 1 für den Ultraschallwandler 15, und die Demodulatoren 2 bzw. 3 derart, daß in jeweils einer Sende-/Empfangsperiode während eines ersten Zeitintervalles der Ultraschallsender und während eines darauffolgenden Zeitintervalles die Demodulatoren aktiv sind. Dazu wird das Trägersignal auf einer Leitung 18 an ein mit seinem Ausgang mit dem Demodulator 2 verbundenes UND-Gatter 11 und auf einer Leitung 19 an ein mit seinem Ausgang mit dem Demodulator 3 verbundenes UND-Gatter 13 geführt. Die UND-Gatter 11 bzw. 13 werden jeweils zum Durchlassen der Trägersignale durch ein erstes Freigabesignal auf einer Leitung 20 bzw. durch ein zweites Freigabesignal auf einer Leitung 21 freigegeben, die jeweils von der Sende-/Empfangssteuerschaltung 8 erzeugt werden. Liegen das erste bzw. das zweite Freigabesignal an dem UND-Gatter 11 bzw. 13 an, gelangt das Trägersignal zu den Demodulatoren 2 bzw. 3, so daß die entsprechenden Dopplersignale erzeugt werden können. Weiterhin aktiviert die Sende-/Empfangssteuerschaltung 8 durch ein drittes Freigabesignal auf einer Leitung 22 die Treiberschaltung 1 für den Ultraschallwandler 15 derart, daß während eines vorbestimmten Zeitintervalles zu Beginn jeder Sende-/Empfangsperiode Ultraschallimpulse ausgesandt werden.

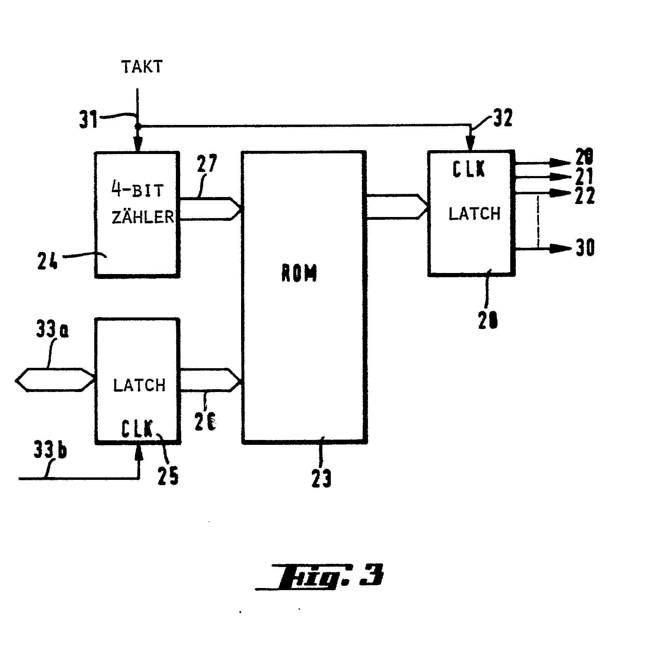

In Figur 3 ist ein Beispiel für eine Sende-/Empfangssteuerschaltung 8 näher erläutert. In einem Lesespeicher (ROM) 23 sind eine Vielzahl von Datenworten gespeichert, deren einzelne Datenbits zur Aktivierung des Ultraschallsenders 1, 15 und der Demodulatoren 2 bzw. 3 im Nutzkanal bzw. im Vergleichskanal verwendet werden. Bei Verwendung eines 4k × 8 ROM werden die oberen acht Adressbits entsprechend den jeweils neu einzustellenden Empfangsfenstern von einem Mikroprozessor (nicht dargestellt) über die mit dem Mikroprozessor verbundenen Busleitungen 33, ein Latch 25 und die Adresseneingänge 26 zur Tiefenselektion angesprochen, während die unteren vier Bits von einem Zähler 24 über die Adresseneingänge 27 angesprochen werden und den jeweiligen Zeitablauf steuern. Falls entsprechend dem dargestellten Ausführungsbeispiel ein 4-Bit Zähler verwendet wird, wird bei jedem neuen Zählerstand ein neues Datenwort mit einer Länge von acht Bit aus dem ROM über ein Latch 28 ausgelesen. Die seriellen Datenflüsse an den Ausgängen 22, 20 bzw. 21 aktivieren jeweils den Ultraschallsender, den Demodulator 2 im Nutzkanal und den Demodulator 3 im Vergleichskanal. Die restlichen fünf Datenleitungen, beispielsweise die Leitung 30, können bei Bedarf für weitere Aufgaben, wie z.B. Anzeigesteuerungen, benutzt werden. Der Takteingang des Latch 25 ist über die Leitung 33b mit dem Steuerbus des Mikroprozessors verbunden und dem Zähler 24 und dem Latch 28 wird auf Leitungen 31 bzw. 32 ein Taktsignal mit einer Frequenz von 51,2 kHz zugeführt. Bei einem 4-Bit Zähler wird somit ein Sende-Empfangszyklus in 16 Zeitintervalle aufgeteilt. Für eine Länge des Sende-/Empfangszyklus von 312,5 Mikrosekunden ergeben sich somit einzelne Zeitbereiche von je 19,5 Mikrosekunden, die einer Tiefenauflösung von etwa 1,5 Zentimetern entsprechen. Die untersuchten Tiefenbereiche im Nutzkanal und im Vergleichskanal können also unabhängig voneinander unter Steuerung durch den Mikroprozessor in Stufen von etwa 1,5 cm (bzw. Vielfachen davon) verstellt werden.

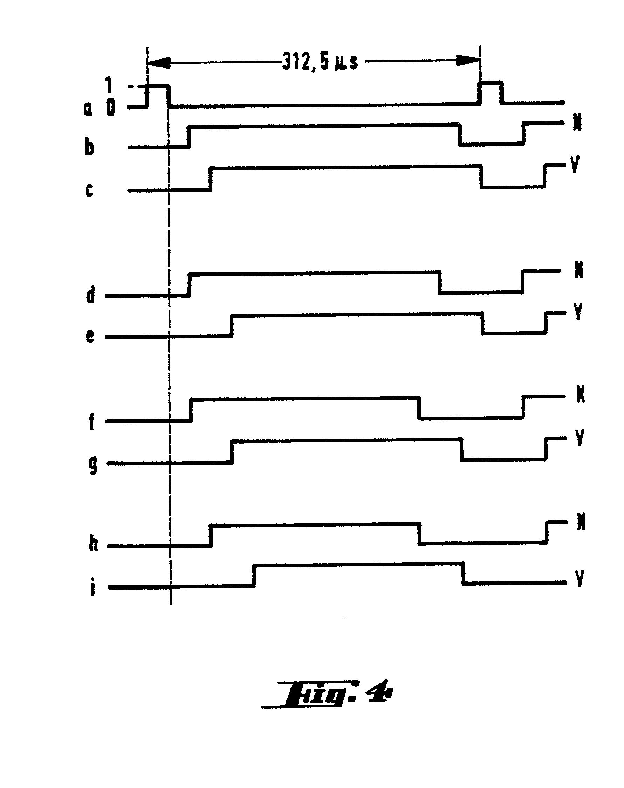

Der zeitliche Verlauf der Signale zum Aktivieren der Treiberschaltung 1 und der Demodulatoren 2 bzw. 3 ist in den Figuren 4a bis 4i an einem Beispiel veranschaulicht. In Figur 4a sind die Impulse zum Erregen der Treiberschaltung 1 dargestellt. In dem vorliegenden Ausführungsbeispiel treten sie mit einer Wiederholfrequenz von 3,2 kHz auf und haben jeweils eine Länge von 19,5 Mikrosekunden. In den Figuren 4b bis 4i sind vier Suchschritte zum Lokalisieren der Tiefe des fötalen Herzens veranschaulicht, wobei jeder Suchschritt während einer anderen Herzschlagsperiode ausgeführt wird. Die Steuersignale für den Nutzkanal sind mit N und die Steuersignale für den Vergleichskanal sind mit V gekennzeichnet. Eine ansteigende Flanke der Steuersignale entspricht dabei dem Beginn der Aktivierung und eine abfallende Flanke dem Ende der Aktivierung des jeweiligen Demodulators. Auf diese Weise wird jeweils ein Empfangsfenster definiert, das entsprechend der Fortpflanzungsgeschwindigkeit der Ultraschallsignale einem bestimmten Tiefenbereich im untersuchten Körper entspricht. Dabei entspricht die linke Seite im dargestellten Zeitverlauf der oberen, nahe am Ultraschallwandler 15 befindlichen Grenze des untersuchten Körperbereiches und die rechte Seite entspricht der unteren Grenze des untersuchten Bereiches innerhalb des Körpers der Schwangeren.

Gemäß Figur 4b wird nach dem Abklingen eines Sendeimpulses (nach Auftreten der abfallenden Flanke des in Figur 4a dargestellten Steuersignales) der Demodulator 2 im Nutzkanal für eine bestimmte Zeitspanne aktiviert, die höchstens so lang ist wie der zeitliche Abstand zweier aufeinanderfolgender Sendeimpulse, im vorliegenden Beispiel 312,5 Mikrosekunden. Wie in Figur 4c dargestellt, wird der Demodulator 3 im Vergleichskanal eine bestimmte Zeitspanne nach der Aktivierung des Demodulators 2 im Nutzkanals aktiviert und eine bestimmte Zeitspanne nach der Beendigung der Aktivierung des Demodulators 2 gesperrt. Die Zeitfenster, während derer der Nutzkanal bzw. der Vergleichskanal zum Erzeugen von Dopplersignalen freigegeben sind, überlappen sich also zu einem großen Teil. Aus dem Amplitudenvergleich der Ausgangssignale der Hüllkurvendemodulatoren 6 und 7 ergibt sich unmittelbar eine Tendenz für die Richtung, in der sich das fötale Herz befindet, bzw. ein Gradient, der die weitere Suchrichtung angibt.

Im vorliegenden Beispiel ist angenommen, daß die Amplitude des Hüllkurvensignales im Nutzkanal größer ist als im Vergleichskanal. Infolgedessen wird im nächsten Suchschritt (vgl. Figuren 4d und 4e) bei einer neuen Herzschlagsperiode das Zeitfenster für den Nutzkanal an der Seite verkleinert, an der das Zeitfenster für den Vergleichskanal über das Zeitfenster für den Nutzkanal hinausreicht. Gleichzeitig wird gemäß Figur 4e das Zeitfenster für den Vergleichskanal um denselben Betrag wie das Fenster für den Nutzkanal gekürzt und das gesamte Fenster wird um einen bestimmten Betrag versetzt. Ist nun beispielsweise die Amplitude des im Nutzkanal gewonnenen Hüllkurvensignales größer als die Amplitude des im Vergleichskanal gewonnenen Hüllkurvensignales, wird im nächsten Suchschritt (Figuren 4f, 4g) das Vergleichsfenster an der Seite verkleinert, an der das Zeitfenster für den Vergleichskanal über das Zeitintervall für den Nutzkanal hinausreicht. Das Vergleichsfenster wird ebenfalls an derselben Seite um denselben Betrag gekürzt. Als Ergebnis erhält man die in den Figuren 4f und 4g gezeigten Fenstereinstellungen. Unter der Annahme, daß nun der Vergleichskanal eine größere Amplitude für das Hüllkurvensignal liefert als der Nutzkanal, wird das Fenster für den Nutzkanal an der Seite gekürzt, die über das Ende des Fensters für den Vergleichskanal hinausragt. Das Ergebnis ist in den Figuren 4h und 4i gezeigt.

In dem hier dargestellten Beispiel werden aus Gründen der Übersichtlichkeit nur die Empfangsfenster variiert, während das Sendefenster konstant auf 19,5 Mikrosekunden gehalten wird. Die Signalqualität könnte jedoch für jeden selektierten Bereich dadurch verbessert werden, daß das Sendefenster vergrößert und das Empfangsfenster um den gleichen Betrag verkürzt würde.

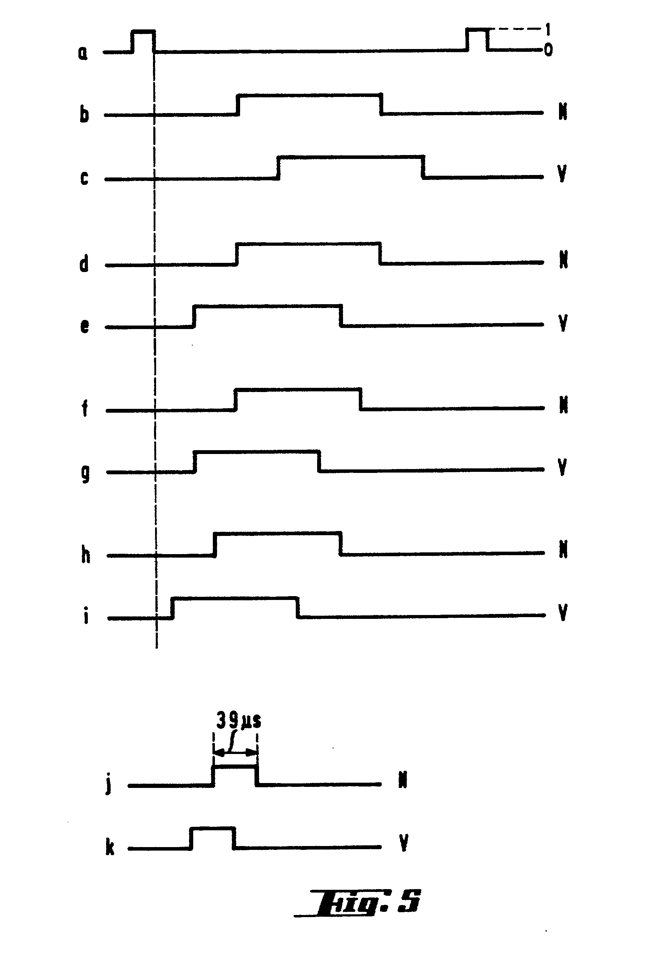

Anhand der Figuren 5a bis 5k wird nachfolgend ein weiteres Beispiel des Suchverfahrens zum Auffinden des fötalen Herzens erläutert. Dieser Teil des Suchverfahrens wird insbesondere dann angewandt, wenn der Fötus sich im Mutterleib entgegen der augenblicklichen Suchrichtung bewegt und so die Möglichkeit besteht, daß das Herzschlagssignal verlorengeht.

In Figur 5a ist die zeitliche Aufeinanderfolge zweier Sendeimpulse dargestellt. Die Figuren 5b bzw. 5c zeigen das Fenster für den Nutzkanal bzw. für den Vergleichskanal nach einigen vorhergehenden, nicht dargestellten Suchschritten. Es sei angenommen, daß keines der Empfangsfenster sich an den Grenzen des Suchbereiches (ansteigende oder abfallende Flanke eines Sendeimpulses) befinde und daß das Hüllkurvensignal im Nutzkanales eine größere Amplitude habe als das im Vergleichskanal. Als Folge davon befindet sich in dem darauffolgenden, in den Figuren 5d und 5e dargestellten Suchschritt das Fenster für den Nutzkanal weiterhin an der bisherigen Position, während das Fenster für den Vergleichskanal nunmehr auf der anderen Seite des Nutzkanalfensters über dieses hinausragt. Die Längen der Fenster bleiben dabei unverändert. In dem in den Figuren 5d und 5e dargestellten Beispiel ist angenommen, daß das Dopplersignal im Vergleichskanal stärker ist als im Nutzkanal. Daraufhin wird das Zeitfenster im Nutzkanal im folgenden Schritt gemäß Figuren 5f und 5g an der Seite gekürzt, welche in Richtung des Versatzes des vorhergehenden Vergleichskanalfensters mit dem kleineren Dopplersignal liegt. Die gleiche Reaktion wäre dann erfolgt, wenn der Nutzkanal nach Figur 5d wieder das stärkere Signal lieferte, der Unterschied zum Vergleichskanal jedoch nicht so groß wie bei 5b und 5c wäre. In Figur 5d ist ein Suchschritt dargestellt der sich ergibt, wenn das Signal im Vergleichssignal plötzlich sehr viel größer ist als im Nutzkanal (etwa um einen Faktor 1,25), beispielsweise aufgrund einer plötzlichen Lageänderung des Herzens. In dem dargestellten Suchschritt wird die Fenstergröße beibehalten, das Fenster wird jedoch als ganzes in die Richtung verschoben, in welche der Versatz des Vergleichsfensters zeigt. Fensterverkürzungen erfolgen so lange, bis eine in Figur 5j und 5k dargestellte minimale vorbestimmte Fensterweite erreicht ist, die eine optimale Tiefenselektion gewährleistet. Eine Verschiebung der Fenster ist auch weiterhin möglich.

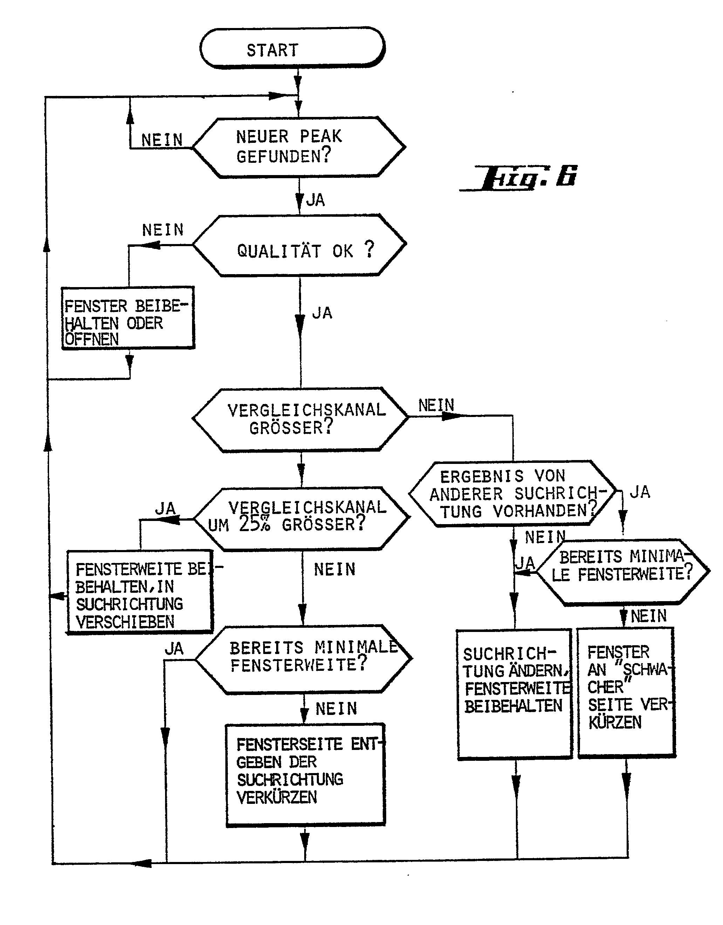

In Figur 6 wird das Suchverfahren zum Lokalisieren der Tiefe des fötalen Herzens anhand eines Flußdiagrammes beschrieben. Die nachfolgend beschriebenen Schritte können dabei programmgesteuert mit Hilfe eines Mikroprozessors ausgeführt werden.

Zunächst wird überprüft, ob im Nutzkanal ein neuer Spitzenwert aufgetreten ist, der einem Herzschlagssignal entsprechen könnte. Wird ein solcher Spitzenwert festgestellt, wird anschließend überprüft ob die Signalqualität ausreichend ist, so daß eine Weiterverarbeitung möglich ist.

Ein Kriterium für die Signalqualität kann beispielsweise abgeleitet werden aus der Autokorrelation des Ausgangssignales des Hüllkurvendemodulators. Die Qualität bestimmt, ob das Empfangsfenster verkleinert, beibehalten oder vergrößert wird. Anschließend beginnt das Verfahren von neuem. Mit dieser Maßnahme sollen "verlorengegangene" Signalquellen wiedergefunden werden oder eine Neuplazierung des Ultraschallwandlers 15 berücksichtigt werden.

Bei ausreichender Signalqualität wird anschließend geprüft, ob die Amplitude des Hüllkurvensignales im Vergleichskanal größer als im Nutzkanal ist. Trifft dies nicht zu, muß zunächst geprüft werden, welches Ergebnis die andere Suchrichtung liefert. Ist dies in einem vorhergehenden Suchschritt noch nicht erfolgt, und auch nicht wie nach Figur 4b zu erwarten, wird die Suchrichtung geändert, die Fensterbreite wird jedoch beibehalten und das Verfahren beginnt wieder am Anfang mit der Überprüfung, ob ein neuer Spitzenwert gefunden wurde. Falls ein Vergleich mit einer anderen Suchrichtung bereits erfolgt ist, wird zuerst überprüft, ob bereits die minimale Fensterweite erreicht ist. Wenn ja, wird ebenfalls die Suchrichtung geändert und das Verfahren beginnt wieder am Anfang. Wenn nein, wird das Fenster an der Seite verkürzt, die in Richtung des Versatzes des (schwächeren) Vergleichskanals liegt und das Verfahren beginnt wieder am Anfang (vergleiche Figuren 5f,5g).

Wenn das Dopplersignal im Vergleichskanal größer ist als im Nutzkanal, wird als nächstes überprüft, ob das Signal im Vergleichskanal wesentlich größer als im Nutzkanal ist, wobei im vorliegenden Beispiel das Entscheidungskriterium ist, ob das Signal im Vergleichskanal das Signal im Nutzkanal um mehr als 25% übersteigt. Falls dies zutrifft, wird die Fensterweite beibehalten und das Fenster wird in Suchrichtung verschoben. Wenn das Signal im Vergleichskanal um weniger als 25% von dem Signal im Nutzkanal abweicht, wird für den Fall, daß bereits die minimale Fensterweite erreicht ist, das Verfahren wieder von neuem gestartet. Ist die minimale Fensterweite noch nicht erreicht, wird die Fensterseite entgegen der Suchrichtung verkürzt und es wird wieder von vorne begonnen.

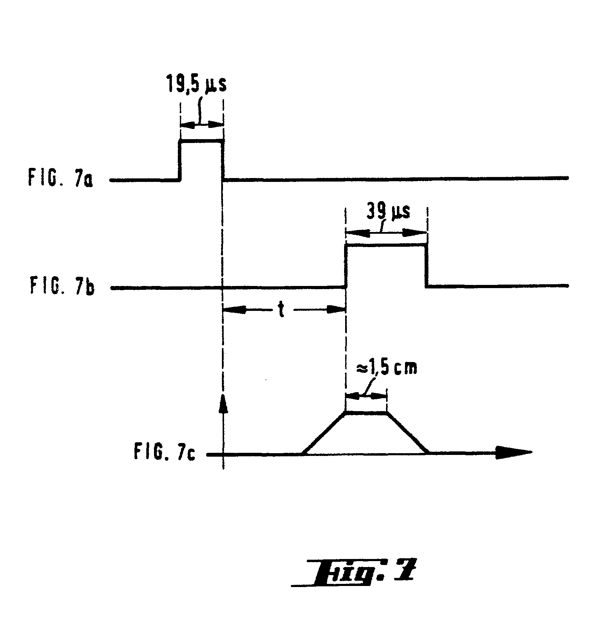

In Figur 7c ist der Empfindlichkeitsbereich der Herzschlgsmessung für den eingeregelten Zustand nach Auffinden der Tiefe des fötalen Herzens dargestellt. Der in Figur 7a dargestellte Sendeimpuls hat eine Länge von 19,5 Mikrosekunden und das Empfangsfenster ist gemäß Figur 7b im eingeregelten Zustand für eine Dauer von 39 Mikrosekunden geöffnet. In Figur 7c ist die aus diesem Empfangsfenster sich ergebende Empfindlichkeit in 7c ist die aus diesem Empfangsfenster sich ergebende Empfindlichkeit in Abhängigkeit von der Tiefe dargestellt. Die Empfindlichkeit ist über einen Bereich von 1,5 cm im wesentlichen konstant und fällt an den Flanken weitgehend linear ab.

Es versteht sich, daß der beschriebene Ablauf zum Eingrenzen des Empfindlichkeitsbereiches auf das fötale Herz nicht der einzig mögliche ist. Wesentlich für die Erfindung ist, daß wenigstens zwei Kanäle vorhanden sind, die infolge ihrer unterschiedlichen zeitlichen Aktivierung Dopplersignale aus verschiedenen Tiefenbereichen liefern, so daß ein Gradient für die Suchrichtung des jeweils nächsten Suchschrittes abgeleitet werden kann.

**Patentansprüche**

1. Schaltungsanordnung zum Erfassen der Herzschlagsbewegung, insbesondere der fötalen Herzschlagsbewegung, mit folgenden Einrichtungen:

a) ein Ultraschallsender (1, 15) zum Aussenden von Ultraschallimpulsen einer vorgegebenen Trägerfrequenz jeweils während einer von einer Vielzahl von aufeinanderfolgenden Sende-/Empfangsperioden,

b) ein Ultraschallempfänger (15) zum Empfang der an dem Herzen reflektierten Ultraschallsignale während jeweils einer Sende-/Empfangsperiode, wobei die empfangenen Signale aufgrund des durch die Herzschlagsbewegung hervorgerufenen Dopplereffektes frequenzverschoben sind,

c) wenigstens zwei mit dem Ultraschallempfänger (15) verbindbare Signalverarbeitungskanäle mit jeweils einer Demodulatorschaltung (2; 3) zum Erzeugen von Dopplersignalen,

d) eine Signalverarbeitungseinrichtung (10) zum Ableiten der Frequenz der Herzschlagsbewegung, dadurch gekennzeichnet, daß

e) jeder Signalverarbeitungskanal ein Bandpaßfilter (4; 5) und einen Hüllkurvendemodulator (6; 7) zum Erzeugen der Hüllkurve des jeweiligen Dopplersignales aufweist,

f) eine Amplitudenvergleichsschaltung (9) vorgesehen ist zum Empfangen der Ausgangssignale der Hüllkurvendemodulatoren (6; 7) und zum Vergleichen der Amplituden der Dopplersignale in den verschiedenen Signalverarbeitungskanälen,

g) eine Steuereinrichtung (8) vorgesehen ist, welche mit jeweils einem Eingang der Demodulatorschaltungen (2; 3) verbunden ist zum Aktivieren der Demodulatorschaltungen (2; 3) während jeweils eines Empfangsintervalles nach dem Aussenden von Ultraschallimpulsen zum Erzeugen von Dopplersignalen, wobei die Dauer der ausgesandten Ultraschallimpulse, die Dauer des Empfangsintervalles und die Dauer des Zeitintervalles zwischen einem ausgesandten Ultraschallimpuls und dem Anfang des Empfangsintervalles derart sind, daß die Dopplersignale in den verschiedenen Signalverarbeitungskanälen aus gegeneinander verschobenen, sich überlappenden Tiefenbereichen des zu untersuchenden Körpers stammen,

h) die Steuereinrichtung (8) mit der Amplitudenvergleichsschaltung (9) verbunden ist zum Ableiten der Richtung, in welcher die Amplitude der Dopplersignale zunimmt und damit in der jeweils folgenden Herzschlagsperiode die Tiefenbereiche durch entsprechende Einstellung des Sendebzw. Empfangsintervalles zu verschieben sind, und

i) die Steuereinrichtung (8) die Demodulatorschaltungen (2; 3) während jeweils einer Sende-/Empfangsperiode zu Zeitpunkten entsprechend der in der vorhergehenden Herzschlagsperiode festgestellten Richtung für das zu untersuchende Herz aktiviert, so daß nach einer Anzahl von Herzschlagsperioden der sich ergebende Tiefenbereich im wesentlichen ausschließlich das schlagende Herz umfaßt.

2. Schaltungsanordnung nach Anspruch 1, dadurch gekennzeichnet, daß die Dauer der ausgesandten Ultraschallimpulse konstant gehalten wird und daß die Dauer der Empfangsintervalle veränderbar ist.

3. Schaltungsanordnung nach Anspruch 1, dadurch gekennzeichnet, daß die Dauer der ausgesandten Ultraschallimpulse veränderbar ist und daß die Dauer der Empfangsintervalle konstant gehalten wird.

4. Schaltungsanordnung nach Anspruch 1, dadurch gekennzeichnet, daß sowohl die Dauer der ausgesandten Ultraschallimpulse als auch die Dauer der Empfangsintervalle veränderbar ist.

5. Schaltungsanordnung nach Anspruch 2 oder 4, dadurch gekennzeichnet, daß bei jeder neuen Herzschlagsperiode die Dauer der Empfangsintervalle gegenüber der jeweils vorhergehenden Herzschlagsperiode verkürzt wird.

6. Schaltungsanordnung nach einem der vorhergehenden Ansprüche dadurch gekennzeichnet, daß zwei Signalverarbeitungskanäle vorgesehen sind, von denen der eine zum Ableiten der Frequenz des Herzschlagssignales und der andere als Vergleichskanal beim Einstellen des Tiefenbereiches des zu untersuchenden Herzens verwendet wird.

## Claims

1. Circuit arrangement for detecting heartbeat motion, in particular for detecting fetal heartbeat motion, comprising

a) an ultrasound transmitter (1.15) for launching ultrasound pulses of a given carrier frequency during any one of a plurality of successive transmitting/receiving periods,

b) an ultrasound receiver (15) for receiving the ultrasonic signals reflected by the heart during any transmitting/receiving period, the signals received being frequency-shifted due to the Doppler effect resulting from the heartbeat motion,

c) at least two signal processing channels, which can be connected to the ultrasound receiver (15), equipped with one demodulator circuit (2; 3) each for generating Doppler signals,

d) signal processing means (10) for deriving the frequency of the heartbeat motion, characterized in

e) that each signal processing channel comprises a bandpass filter (4; 5) and an envelope demodulator (6; 7) for generating the envelope of the respective Doppler signal,

f) that an amplitude comparator circuit (9) is provided for receiving the output signals of the envelope demodulators (6; 7) and for comparing the amplitudes of the Doppler signals in the different signal processing channels,

g) that control means (8) connected to one input of each of the demodulator circuits (2; 3) is provided for activating the demodulator circuits (2; 3) during each of the receiving intervals, after ultrasonic pulses have been launched, for generating Doppler signals, the length of the launched ultrasonic pulses, the duration of the receiving intervals and the duration of the time interval between the launched ultrasonic pulse and the beginning of the receiving interval being selected in such a manner that the Doppler signals in the different signal processing channels originate from overlapping depth ranges of the body under examination which are shifted relative to each other,

h) that the control means (8) is connected with the amplitude comparator circuit (9) for deriving the direction in which the amplitude of the Doppler signals increases and in which, accordingly, the depth ranges have to be shifted in the next following heartbeat period by appropriate adjustment of the transmitting or receiving interval, and

i) that the control means (8) activates the demodulator circuits (2; 3) during each transmitting/receiving period at points in time in accordance with the direction determined in the preceding heartbeat period for the heart to be examined, so that after a number of heartbeat periods the depth range obtained includes practically exclusively the beating heart.

2. Circuit arrangement according to claim 1, characterized in that the duration of the launched ultrasonic pulses is kept constant and the duration of the receiving intervals is variable.

3. Circuit arrangement according to claim 1, characterized in that the duration of the launched ultrasonic pulses is variable and the duration of the receiving intervals is kept constant.

4. Circuit arrangement according to claim 1, characterized in that both the duration of the launched ultrasonic pulses and the duration of the receiving intervals are variable.

5. Circuit arrangement according to claim 2 or 4, characterized in that the duration of the receiving intervals is shortened for each new heartbeat period, relative to the respective preceding heartbeat period.

6. Circuit arrangement according to any of the preceding claims, characterized in that two signal processing channels are provided, one of which is used for deriving the frequency of the heartbeat signal, while the other one is used as comparator channel for adjusting the depth range of the heart under examination.

**Revendications**

1. Montage pour détecter les battements cardiaques, notamment les battements cardiaques d'un fœtus, comportant les dispositifs suivants:
   a) un émetteur à ultrasons (1, 15) servant à émettre des impulsions ultrasonores possédant une fréquence porteuse prédéterminée, et ce respectivement pendant une période faisant partie d'une pluralité de périodes successives d'émission/réception,
   b) un récepteur d'ultrasons (15) servant à recevoir les signaux ultrasonores réfléchis sur le cœur, pendant une période respective d'émission/réception, les signaux reçus étant décalés en fréquence en raison de l'effet Doppler provoqué par les battements cardiaques,
   c) au moins deux canaux de traitement des signaux, qui peuvent être reliés au récepteur d'ultrasons (15) et comportent chacun un circuit démodulateur (2, 3) servant à produire des signaux Doppler,
   d) un dispositif (10) de traitement de signaux, servant à dériver la fréquence des battements cardiaques, caractérisé par le fait que
   e) chaque canal de traitement de signaux comporte un filtre passe-bande (4; 5) et un démodulateur (6, 7) de la courbe enveloppe, servant à produire la courbe enveloppe du signal Doppler respectif,
   f) un circuit comparateur d'amplitude (9) est prévu pour la réception des signaux de sortie des démodulateurs (6; 7) de la courbe enveloppe et pour la comparaison des amplitudes des signaux Doppler présents dans les différents canaux de traitement de signaux,
   g) il ist prévu un dispositif de commande (8), qui est relié à une entrée respective des circuits démodulateurs (2; 3) pour l'activation de ces circuits démodulateurs (2; 3), pendant un intervalle respectif de réception après l'émission d'impulsions ultrasonores en vue de la production de signaux Doppler, auquel cas la durée des impulsions ultrasonores émises, la durée des intervalles de réception et la durée de l'intervalle de temps s'étendant entre l'impulsion ultrasonore émise et le début de l'intervalle de réception sont choisies de manière que les signaux Doppler présents dans les différents canaux de traitement des signaux proviennent de zones de profondeur, décalées les unes par rapport aux autres et se chevauchant, du corps à examiner,
   h) le dispositif de commande (8) est relié au circuit comparateur d'amplitude (9) pour l'obtention de la direction, dans laquelle l'amplitude des signaux Doppler augmente, et dans laquelle, par conséquent, pendant la période respectivement suivante des battements cardiaques, les zones de profondeur doivent être décalées au moyen d'un réglage correspondant de l'intervalle d'émission ou de réception, et
   i) le dispositif de commande (8) active les circuits démodulateurs (2; 3) respectivement pendant une période d'émission/réception, à des instants correspondants à la direction déterminée pendant la période précédente des battements cardiaques, pour le cœur à examiner, de sorte qu'au bout d'un certain nombre de périodes des battements cardiaques, la zone de profondeur obtenue englobe essentiellement exclusivement le cœur vivant.

2. Montage selon la revendication 1, caractérisé en ce que la durée des impulsions ultrasonores émises est maintenue constante et que la durée des intervalles de réception est modifiable.

3. Montage selon la revendication 1, caractérisé en ce que la durée des impulsions ultrasonores émises est modifiable et que la durée des intervalles de réception est maintenue constante.

4. Montage selon la revendication 1, caractérisé en ce qu'aussi bien la durée des impulsions ultrasonores émises que la durée des intervalles de réception est modifiable.

5. Montage selon la revendications 2 ou 4, caractérisé en ce que, lors de chaque nouvelle période des battements cardiaques, la durée des intervalles de réception est raccourcie par rapport à la période respectivement précédente des battements cardiaques.

6. Montage selon l'une des revendications précédentes, caractérisé en ce qu'il est prévu deux canaux de traitement des signaux, dont l'un est utilisé pour l'obtention de la fréquence du signal du battement cardiaque et dont l'autre est utilisé en tant que canal de comparaison lors du réglage de la zone en profondeur du cœur à examiner.

*Fig. 1*

TRÄGERSIGNAL

SENDE-/ EMPFANGS-STEUERUNG

DEMODU-LATOR

BANDPASS-FILTER

HÜLLKURVEN DEMODU-LATOR

DEMODU-LATOR

BANDPASS-FILTER

HÜLLKURVEN DEMODULA-TOR

AMPLIT.-VERGLEICH

SIGNAL-VERARBEI-TUNG

*Fig. 2*

EP 0 204 192 B1

**Fig. 3**

Fig. 4

Fig. 5

Fig. 6

FIG. 7a

FIG. 7b

FIG. 7c

19,5 μs

39 μs

t

≈1,5 cm

Fig. 7